# EUROPÄISCHE PATENTSCHRIFT

(11) **EP 2 382 924 B1**
(45) Veröffentlichungstag und Bekanntmachung des Hinweises auf die Patenterteilung: **25.07.2018**
(21) Anmeldenummer: 11001850.4
(22) Anmeldetag: 05.03.2011
(51) Int. Cl.: A61B 17/00, A61B 17/225, A61N 7/00

(54) **STOSSWELLENTHERAPIEGERÄT FÜR DIE EXTRAKORPORALE STOSSWELLENTHERAPIE**
Shock wave therapy device for extracorporeal shock wave therapy
Appareil de thérapie à ondes de choc pour la thérapie à ondes de choc extracorporelle

(30) Priorität: 29.04.2010 DE 102010018707
(43) Veröffentlichungstag der Anmeldung: 02.11.2011
(73) Patentinhaber: Richard Wolf GmbH, 75438 Knittlingen (DE)
(72) Erfinder: Bauer, Edgar, 76703 Kraichtal (DE); Krauß, Werner, 75438 Knittlingen (DE)
(74) Vertreter: Hemmer, Arnd

(56) Entgegenhaltungen:
- EP-A1- 0 359 863
- EP-A2- 0 330 816
- EP-A2- 0 449 138
- EP-A2- 1 698 287
- DE-A1-102005 031 117
- DE-A1-102006 024 424
- DE-A1-102007 030 066
- US-A1- 2004 167 397
- US-A1- 2004 179 332
- US-A1- 2009 281 464

## Beschreibung

Die Erfindung betrifft ein Stoßwellentherapiegerät für die extrakorporale Stoßwellentherapie mit den im Oberbegriff des Anspruchs 1 angegebenen Merkmalen.

Die extrakorporale Stoßwellentherapie (ESWT) wird seit mehreren Jahren erfolgreich in der Orthopädie zur nicht invasiven Behandlung des knochennahen Weichteilgewebes eingesetzt. Standardindikation sind beispielsweise Kalkschulter, Tennis- und Golferellenbogen, Patellaspitzensyndrom, Tibiakantensyndrom, Archillodynie und Fersensporn. Weitere Indikationen sind so genannte Triggerpunkte in Muskeln. Darüber hinaus kommt die Stoßwellentherapie bei neueren Indikationen, wie zum Beispiel Wundbehandlung, Cellulite, Fettgewebe oder bei dem chronischen Beckenbodensyndrom zum Einsatz, wobei bei diesen Therapien großflächige Gewebeareale therapiert werden.

Bei den Therapien werden an einem oder mehreren Punkten eines A-reals Stoßwellen appliziert, wobei üblicherweise mit Stoßwellen einer geringeren Intensität begonnen wird und dann im Verlauf der Behandlung die Intensität gesteigert werden kann, da die Stoßwellen eine sedierende Wirkung haben. Hierzu sind während der Behandlung stetig Veränderungen der Einstellungen am Stoßwellengenerator erforderlich.

EP 0 449 138 A2 offenbart ein Ultraschallbehandlungssystem. Bei diesem System können Frequenz und Stärke der Ultraschallimpulse voreingestellt werden und dann der voreingestellte Behandlungsplan ausgeführt werden. Dann wird für eine größere Anzahl von Ultraschallimpulsen mit konstanter Leistung und konstanter Frequenz abgegeben.

DE 10 2005 031 117 A1 offenbart ein Verfahren zum Bestimmen eines Betriebsparameters einer Stoßwellenquelle gemäß dem ein Betriebsparameter in Abhängigkeit einer vor oder während der Behandlung ermittelten Kenngröße eingestellt wird. Der Betriebsparameter kann die Amplitude oder die Frequenz der Stoßwellen sein.

DE 10 2007 030 066 A1 offenbart ein Verfahren zur Vorbereitung einer extrakorporalen Stoßwellentherapie, bei welchem mit Hilfe eines MR-Bildgebungssystems das Zielgebiet im Gewebe lokalisiert wird und der Fokuspunkt richtig zugeordnet wird.

DE 10 2006 024 424 A1 betrifft eine Fernsteuerung für eine medizinische Anlage beispielsweise bei der Ultraschallstoßwellenbehandlung. Über die Fernbedienung können Betriebsparameter des medizinischen Gerätes eingestellt werden.

EP 0 330 816 A2 offenbart eine Vorrichtung zur Erzeugung von Ultraschallsignalen, bei welcher Signalfolgen abgegeben werden können, wobei die Amplitude zwischen einzelnen Signalfolgen geändert werden kann.

US 2004/0179332 A1 und US 2004/0167397 A1 offenbaren portable Ultraschallgeräte.

Es ist Aufgabe der Erfindung ein verbessertes Stoßwellentherapiegerät zu schaffen, mit welchem der Behandlungsablauf zum einen vereinfacht werden kann und zum anderen eine verbesserte Anpassung des Behandlungsablaufes an die Bedürfnisse des einzelnen Patienten sowie die jeweilige Indikation möglich wird.

Die Aufgabe wird durch ein Stoßwellentherapiegerät mit den in Anspruch 1 angegebenen Merkmalen gelöst. Bevorzugte Ausführungsformen ergeben sich aus den Unteransprüchen, der nachfolgenden Beschreibung sowie den beigefügten Figuren.

Das erfindungsgemäße Stoßwellentherapiegerät für die extrakorporale Stoßwellentherapie weist wie bekannte Geräte eine Stoßwellenquelle und eine Steuereinrichtung zur Ansteuerung der Stoßwellenquelle auf. Über die Steuereinrichtung werden beispielsweise die einzelnen Stoßwellen ausgelöst und die Intensität eingestellt.

Erfindungsgemäß ist die Steuereinrichtung mit einem Programmmodul versehen, welches weitere Voreinstellungen ermöglicht. So ist das Programmmodul dafür vorgesehen, vordefinierte Stoßwellenfolgen zu erzeugen. D. h. die Steuereinrichtung aktiviert die Stoßwellenquelle derart, dass sie automatisch eine vordefinierte Stoßwellenfolge bestehend aus einer Vielzahl von einzelnen Stoßwellen abgibt. Die vorbestimmte Folge bzw. Abfolge von Stoßwellen ist in dem Programmmodul gespeichert oder an diesem voreinstellbar. Beim Auslösen bzw. Aktivieren der Stoßwellenquelle steuert die Steuereinrichtung die Stoßwellenquelle dann so, dass diese die vorbestimmte Abfolge von Stoßwellen abgibt. Auf diese Weise kann die Behandlung erheblich vereinfacht und auch besser auf die individuellen Bedürfnisse der jeweiligen Indikation oder des jeweiligen Patienten zugeschnitten werden. So ist es beispielsweise möglich, für bestimmte Therapieformen oder auch bestimmte Patienten vorbestimmte definierte Abfolgen von einzelnen Stoßwellen in dem Programmmodul zu hinterlegen bzw. einzustellen. Während der Behandlung sind dann keine ständigen Veränderungen der Einstellungen am Therapiegerät erforderlich, da beispielsweise Intensitäts- und Frequenzänderungen über das Programmmodul voreingestellt werden, so dass die Steuereinrichtung dann über das Programmmodul automatisiert die vorgegebene Stoßwellenfolge erzeugt.

Die Steuereinrichtung ist so ausgebildet, dass in dem Programmmodul zumindest eine vorbestimmte Abfolge einzelner Stoßwellen unterschiedlicher Intensität gespeichert oder einstellbar ist. Dies ermöglicht es, an dem Programmmodul eine Abfolge einzelner Stoßwellen voreinzustellen bzw. zu definieren, welche unterschiedliche Intensitäten aufweisen. So ist es beispielsweise möglich, in der Abfolge die Intensität automatisiert zu steigern, so dass die sedierende Wirkung der Stoßwellen ausgenutzt wird, um Stoßwellen höherer Intensität möglichst schmerzfrei applizieren zu können. Durch die Möglichkeit die Intensitätsverläufe in einer Abfolge einer Vielzahl einzelner Stoßwellen über das Programmmodul voreinzustellen, wird die eigentliche Behandlung dann vereinfacht, da Einstellungen der Intensität während der Behandlung dann nicht mehr manuell vorgenommen werden müssen.

Ferner ist das Programmmodul so ausgebildet sein, dass zumindest eine vorbestimmte Abfolge einzelner Stoßwellen unterschiedlicher Frequenz, Impulsdauer und/oder mit unterschiedlich langen Pausen gespeichert oder einstellbar ist. So lässt sich beispielsweise die Gesamtimpulsdauer einer Vielzahl aufeinanderfolgender Einzelstoßwellen voreinstellen. In einer Abfolge derartiger Stoßwellen kann darüber hinaus die Länge und Zahl der Pausen zwischen den einzelnen Impulsen oder Stoßwellen voreingestellt und insbesondere über die Gesamtabfolge von Einzelstoßwellen in vordefinierter Weise variiert werden. Die Pausen dienen zur Erholung bzw. Regeneration, um einer Überanspruchung des Gewebes vorzubeugen. So kann beispielsweise nach einer bestimmten Anzahl von Stoßwellen eine etwas längere Pause vorgesehen werden, bevor wieder eine weitere Anzahl von Stoßwellen automatisiert appliziert wird. Durch Veränderung der Frequenz wird die

Zahl der einzelnen Stoßwellen, welche pro Zeiteinheit abgegeben werden, variiert. Bevorzugt erfolgt die Ausbildung längerer Gesamtimpulse und dazwischenliegender Pausen durch eine Amplitudenmodulation einer zugrundeliegenden Grundfrequenz von Einzelstoßwellen.

Die Steuereinrichtung ist darüber hinaus weiter bevorzugt so ausgebildet, dass sie zur Erzeugung unterschiedlicher Intensitätsverläufe in der Abfolge einzelner Stoßwellen ausgebildet ist. Dies kann in der vorangehend beschriebenen Weise erfolgen, indem aufeinanderfolgende Einzelstoßwellen, welche mit einer vorbestimmten Frequenz abgegeben werden, in ihrer Amplitude, d. h. Intensität, so geändert werden, dass aus den Einzelimpulsen zusammengesetzte Gesamtimpulse mit sich ändernder Intensität gebildet werden. So kann beispielsweise ein sinusförmiger, sägezahnförmiger oder rechteckförmiger Impulsverlauf über die Gesamtabfolge der Stoßwellen realisiert werden. Auch der Intensitätsverlauf der Abfolge von Stoßwellen ist vorzugsweise über das Programmmodul Speicher- bzw. voreinstellbar. Dabei können auch Variationen des Intensitätsverlaufes über die Gesamtabfolge speicher- bzw. voreinstellbar sein.

Das Programmmodul ist weiter bevorzugt mit einem Speicher ausgestattet, in welchem mehrere vorbestimmte Abfolgen von Stoßwellen gespeichert oder speicherbar sind. Dabei können diese Abfolgen einzelner Stoßwellen die vorangehend beschriebenen Variationen in Intensität, Frequenz, Impuls- und/oder Pausendauer aufweisen. Ein solcher Speicher ermöglicht es, Abfolgen zu speichern, welche für unterschiedliche Anwendungen bzw. Indikationen vorgesehen sind. So kann der Anwender dann für die jeweilige Indikation eine vordefinierte Abfolge von Stoßwellen an dem Programmmodul auswählen und diese zur Anwendung bringen. Alternativ oder zusätzlich kann das Programmmodul so ausgebildet sein, dass einmal manuell eingestellte Abfolgen von Stoßwellen für spätere Behandlungen individuell speicherbar sind. Auch eine Speicherung von Abfolgen von Stoßwellen, welche patientenspezifisch eingestellt oder ausgewählt sind, ist denkbar. Auf diese Weise wird dann bei der Behandlung die Einstellung des Therapiegerätes deutlich vereinfacht, da aus einer Anzahl vordefinierter bzw. zuvor gespeicherter Folgen bzw. Abfolgen von Stoßwellen nur die jeweils gewünschte ausgewählt werden muss und dann nach Platzierung des Therapiekopfes bzw. der Stoßwellenquelle aktiviert werden muss. Weitere Einstellungen des Therapiegerätes sind dann vorzugsweise nicht mehr erforderlich.

Ferner ist das Programmmodul so ausgestaltet, dass zumindest eine Abfolge einzelner Stoßwellen gespeichert oder einstellbar ist, deren Frequenz, Intensität, Pausen- und Impulsdauer zyklisch variieren. So ist es beispielsweise möglich, Abfolgen von Stoßwellen voreinzustellen, welche zyklisch die Intensität der Stoßwellen steigern und nach einer Anzahl von Einzelimpulsen eine gewisse Pause oder Zeitdauer mit Stoßwellen geringerer Intensität vorsehen, welche als Erholungsphase für das Gewebe vorgesehen ist.

Zweckmäßigerweise ist an dem Stoßwellentherapiegerät zumindest eine Eingabeeinrichtung vorgesehen, mittels welcher eine vorbestimmte Abfolge von Stoßwellen an dem Programmmodul auswählbar oder einstellbar ist. Die Eingabeeinrichtung kann beispielsweise in Form von Eingabetasten, Dreh- und/oder Schieberegler, einer Computermaus, eines Touchpads oder in anderer bekannter Weise ausgebildet sein. Über die Eingabeeinrichtung kann der Benutzer entweder gespeicherte Abfolgen von Stoßwellen an dem Programmmodul auswählen oder gegebenenfalls auch neue Abfolgen an dem Programmmodul einstellen und abspeichern oder auch zuvor eingestellte oder gespeicherte Abfolgen variieren oder verändern. Die Steuereinrichtung ist entsprechend ausgestaltet, um über die Eingabeeinrichtung entsprechende Einstellungen zu ermöglichen.

Besonders bevorzugt weist die Eingabeeinrichtung einen berührungsempfindlichen Bildschirm auf, auf dem beispielsweise mittels eines Eingabestiftes oder auch mit den Fingern die gewünschten Einstellungen vorgenommen werden können. Hierdurch wird eine sehr einfache Bedienung ermöglicht und gleichzeitig die Zahl der Bedienelemente reduziert.

Besonders bevorzugt ist die Steuereinrichtung dabei derart ausgebildet, dass der Kurvenverlauf einer Abfolge von Stoßwellen durch Bewegen einer Kurve auf dem Bildschirm voreinstellbar ist. Dies kann beispielsweise dadurch erfolgen, dass bestimmte Punkte der Kurve manuell markiert werden und dann so verschoben werden, dass ein gewünschter Kurvenverlauf erzeugt wird. Das Markieren und Verschieben kann beispielsweise über ein Eingabeelement wie eine Computermaus oder bei Verwendung eines berührungsempfindlichen Bildschirmes auch direkt auf dem Bildschirm mittels eines Eingabestiftes oder idealerweise direkt mit den Fingern erfolgen. Auf diese Weise wird es sehr einfach, eine voreingestellte Abfolge von Stoßwellen mit gewünschtem Intensitätsverlauf und gegebenenfalls Pausen am Programmmodul voreinzustellen.

Gemäß einer weiteren bevorzugten Ausführungsform weist die Steuereinrichtung einen Lernmodus auf, in welchem eine Behandlungssequenz mit einer speziellen Abfolge einzelner Stoßwellen erlernt und in dem Programmmodul gespeichert werden kann. So kann der Lernmodus so ausgestaltet sein, dass zunächst eine Abfolge einzelner Stoßwellen manuell eingestellt werden kann und dann in dem Programmmodul als vordefinierte Stoßwellenfolge gespeichert wird.

Das erfindungsgemäße Stoßwellentherapiegerät, wie es vorangehend beschrieben wurde, ermöglicht ein neuartiges Therapieverfahren mittels Stoßwellen. Bei diesem Therapieverfahren können automatisiert Abfolgen vordefinierter Einzelstoßwellen auf das Gewebe appliziert werden. Die Stoßwellenquelle bzw. der Therapiekopf wird dazu an gewünschter Stelle des Körpers platziert und ausgerichtet. Anschließend wird eine zuvor ausgewählte vordefinierte Stoßwellenfolge aktiviert und dann automatisiert von der Stoßwellenquelle auf das Gewebe appliziert, ohne dass bei dieser Anwendung noch weitere manuelle Einstellungen und insbesondere Veränderungen der Parameter während der Applikation der Stoßwellenfolge erforderlich ist. Eine so applizierte vordefinierte Stoßwellenfolge ist bevorzugt so ausgebildet, dass die Intensität der Stoßwellen im Verlauf der Stoßwellenfolge variiert, bevorzugt zunächst schrittweise oder linear bis zu einem Maximum erhöht wird und anschließend wieder sprunghaft, stufenweise oder linear verringert wird, so dass während der Behandlung Stoßwellen stärkerer Intensität sich mit Pausen, in welchen keine oder nur Stoßwellen mit geringerer Intensität appliziert werden, abwechseln. Die Pausen bilden Erholungsphasen für das Gewebe. Dabei können unterschiedliche Intensitätsverläufe, beispielsweise mit rechteckförmigem, sägezahnförmigem oder wellenförmigem Verlauf appliziert werden. Auch kann die Länge der Pausen im Gesamtverlauf variiert werden. So kann beispielsweise nach mehreren längeren Impulsen, welche gegebenenfalls aus einer Vielzahl von Einzelimpulsen gebildet werden, jeweils eine längere Pause vorgesehen werden, um eine Erholung des Gewebes zu ermöglichen. Weitere Aspekte des Verfahrens ergeben sich aus der obigen Beschreibung des Therapiegerätes.

Nachfolgend wird die Erfindung beispielhaft anhand der beigefügten Figuren beschrieben. In diesen zeigt:
- Fig. 1: schematisch den Aufbau eines erfindungsgemäßen Stoßwellentherapiegerätes,
- Fig. 2a - 2d: vier mögliche Intensitätsverläufe einer Stoßwellenfolge,
- Fig. 3a: eine Stoßwellenfolge gemäß einer ersten Variation,
- Fig. 3b: vergrößert den Ausschnitt B in Fig. 3a,
- Fig. 4a: eine zweite Variante einer Stoßwellenfolge,
- Fig. 4b: vergrößert den Ausschnitt B in Fig. 4a,
- Fig. 5a: eine dritte Variante einer Stoßwellenfolge,
- Fig. 5b: den Ausschnitt 1 in Fig. 5a,
- Fig. 5c: den Ausschnitt 2 in Fig. 5a und
- Fig. 6: schematisch die Veränderung des Kurvenverlaufes der Intensität einer Stoßwellenfolge.

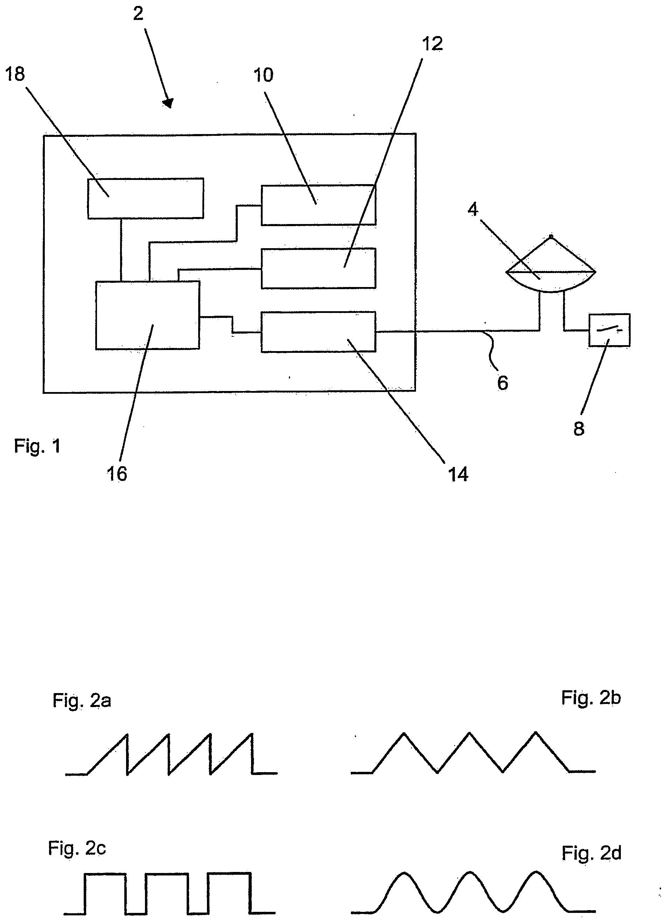

Das in Fig. 1 gezeigte Stoßwellentherapiegerät weist eine Steuereinrichtung 2 und einen Therapiekopf bzw. eine Stoßwellenquelle 4 auf, welche über ein Kabel 6 mit der Steuereinrichtung 2 verbunden ist. An der Stoßwellenquelle 4 ist ein Taster 8 zum Auslösen bzw. Aktivieren von Stoßwellen oder Stoßwellenfolgen angebracht.

Die Steuereinrichtung 2 weist als wesentliche Komponenten eine Eingabeeinrichtung 10, einen Bildschirm 12 und eine HV-Einheit 14 auf, welche die Hochspannungsimpulse für die Stoßwellenquelle 4 erzeugt. In der Steuereinrichtung 2 ist eine zentrale Steuereinheit (CPU) 16 vorgesehen, welche mit dem Bildschirm 12, der Eingabeeinrichtung 10 sowie der HV-Einheit 14 zum Signal- bzw. Informationsaustausch verbunden ist. Erfindungsgemäß ist in der Steuereinrichtung 2 darüber hinaus ein Programmmodul 18 vorhanden. Dieses kann als eigene Hardware oder auch nur als Softwaremodul ausgestaltet sein, welches in der Steuereinrichtung 2, insbesondere dem Programmmodul 18 abläuft. Während bei herkömmlichen Stoßwellentherapiegeräten an der Steuereinrichtung 2 lediglich die Intensität und Impulsdauer eines einzelnen Stoßwellenimpulses voreingestellt werden konnte, welcher dann bei Aktivierung über den Taster 8 von der Stoßwellenquelle 4 ausgesandt wurde, ist es über das erfindungsgemäß vorgesehene Programmmodul 18 möglich, in der Steuereinrichtung 2 vordefinierte Abfolgen bzw. Folgen einer Vielzahl einzelner Stoßwellen im Vorfeld zu definieren. Dabei kann in dieser Abfolge die Intensität, Frequenz, Impulsdauer und/oder mögliche Pausen zwischen einzelnen Stoßwellen variieren. Derartige Stoßwellenfolgen können vordefiniert in dem Programmmodul 18 für unterschiedliche Indikationen abgelegt sein, sodass je nach Einsatzzweck eine vordefinierte Stoßwellenfolge über die Eingabeeinrichtung 10 und den Bildschirm 12 aus dem Speicher des Programmmoduls 18 ausgewählt werden kann. Auch kann es möglich sein, individuelle patientenspezifische Stoßwellenfolgen für spätere Behandlungen in dem Programmmodul 18 zu speichern und entsprechend auszuwählen. Hierzu können über die Eingabeeinrichtung 10 und den Bildschirm 12 entweder vorgegebene Stoßwellenfolgen variiert bzw. verändert und anschließend abgespeichert werden oder aber gegebenenfalls auch neue Stoßwellenfolgen programmiert und anschließend im Programmmodul 18 für spätere Anwendungen gespeichert werden.

Nach Auswahl einer vordefinierten Stoßwellenfolge oder Einstellung einer vordefinierten Stoßwellenfolge an dem Programmmodul 18 wird dann durch Betätigung des Tasters 8 nur noch das Aussenden dieser Stoßwellenfolge gestartet. Anschließend steuert die Steuereinrichtung 2, insbesondere deren zentrale Steuereinheit 16 dann basierend auf den Informationen aus dem Programmmodul 18 die HV-Einheit 14 so, dass die Stoßwellenquelle 4 die Stoßwellen gemäß der vordefinierten Stoßwellenfolge aussendet, ohne, dass hierzu während der Anwendung noch weitere Anpassungen oder Veränderungen erforderlich wären. D. h. erfindungsgemäß ist vorgesehen, dass eine Stoßwellenfolge entweder zuvor in dem Programmmodul 18 gespeichert ist und ausgewählt wird oder aber vor der Applikation in dem Programmmodul 18 voreingestellt wird und dann bei Aktivierung über den Taster 8 von der Steuereinrichtung 2 komplett abgearbeitet wird, so dass die ganze Folge dann automatisiert von der Stoßwellenquelle 4 erzeugt wird. D. h. die vorgegebenen Einzelstoßwellen werden gemäß der vorgegebenen jeweiligen Frequenz, Intensität, Pulsdauer und gegebenenfalls Pausenlänge zwischen den einzelnen Stoßwellen nacheinander von der Stoßwellenquelle 4 automatisch ausgesandt. Durch dieses Verfahren wird es während der Behandlung wesentlich einfacher umfangreiche Stoßwellenvariationen anzuwenden, insbesondere die Intensität und Pausendauer so zu variieren, dass die Intensität schrittweise gesteigert werden kann, um Schmerzen zu verhindern und gegebenenfalls ausreichende Pausen als Erholungsphasen für das Gewebe vorzusehen. Um dies zu erreichen, muss der Anwender während der Anwendung keine weiteren Anpassungen mehr vornehmen.

Insbesondere ist es möglich, den Intensitätsverlauf der Stoßwellen über der Zeit in unterschiedlichen Formen zu variieren. Dabei ist es bevorzugt, dass die Intensität zyklisch ansteigt und dann wieder abfällt. Durch das Ansteigen wird erreicht, dass unter Ausnutzung der sedierenden Wirkung der Stoßwellen die Intensität möglichst schmerzfrei gesteigert werden kann und zum anderen durch die immer wieder erfolgende zyklische Verringerung der Intensität dem Gewebe zwischen den einzelnen Stoßwellenpulsen ausreichen Regenerationsphasen gegeben werden. Fig. 2a zeigt einen sägezahnförmigen Intensitätsverlauf über die Zeit. Fig. 2b zeigt einen Zickzackverlauf, während Fig. 2c einen rechteckigen Verlauf der Intensitätskurve zeigt und Fig. 2d einen wellenförmigen Intensitätsverlauf über die Zeit darstellt. Es ist zu verstehen, dass auch andere Variationen der Intensität über der Zeit Anwendung finden können, auch insbesondere Kombinationen der gezeigten Intensitätsverläufe.

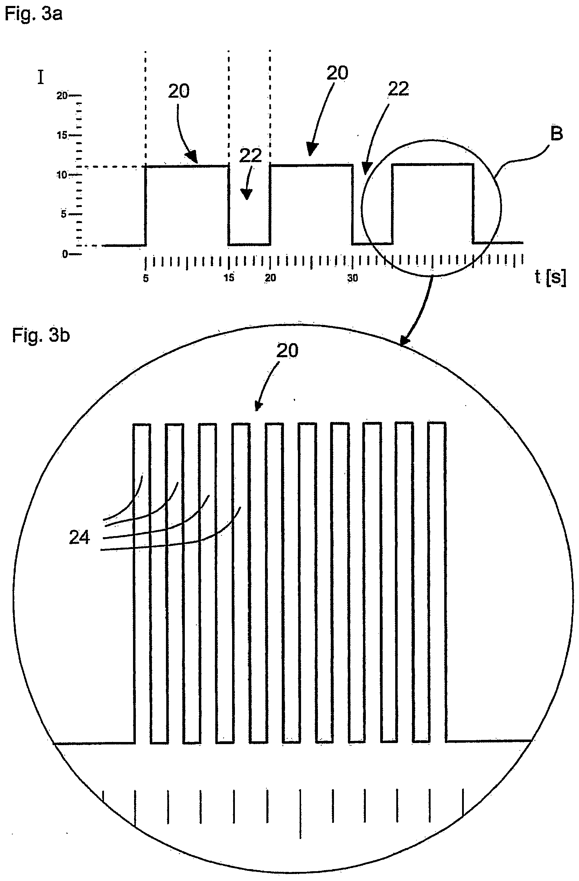

Ein Beispiel eines rechteckförmigen Intensitätsverlaufes wird anhand der Figuren 3a und 3b näher läutert. In Fig. 3a erfolgt die Intensitätsänderung sprunghaft zwischen den Intensitätsstufen 1 und 11, wodurch ein rechteckförmiger Intensitätsverlauf der Stoßwellenfolge erreicht wird. Der rechteckförmige Intensitätsverlauf der Stoßwellenfolge wird bei dem Ausführungsbeispiel wie folgt realisiert. Grundlage ist eine Stoßwellenfolge mit konstanter Frequenz von einem Hertz. Das heißt in Abständen von einer Sekunde wird jeweils eine Stoßwelle ausgesandt. Die Intensität der Stoßwellen wird zwischen zwei Intensitätsstufen variiert, nämlich den Stufen 1 und 11. Zunächst werden bis zum Zeitpunkt von fünf Sekunden Stoßwellen mit der Intensitätsstufe 1 ausgesandt. Am Zeitpunkt fünf Sekunden wird die Intensität sprunghaft auf die Intensitätsstufe 11 erhöht. Anschließend werden, wie in Fig. 3b gezeigt, zehn Einzelstoßwellen mit der höheren Intensität ausgesandt, wodurch ein Stoßwellenimpuls 20 gebildet wird. Anschließend wird nach zehn Sekunden, das heißt zum in Fig. 3a gezeigten Zeitpunkt von fünfzehn Sekunden die Intensität wieder auf die Intensitätsstufe 1 reduziert und diese bei konstanter Stoßwellenfrequenz bis zum Zeitpunkt von zwanzig Sekunden beibehalten. So wird eine Pause 22 mit geringer Intensität der Stoßwellen geschaffen, welche zur Regeneration des Gewebes dient. Anschließend wir die Intensität wieder erhöht und für zehn Sekunden bei einer Stoßwellenfrequenz von einem Hertz beibehalten, sodass ein zweiter Impuls 20 gebildet wird, und so weiter. Der in Fig. 3a gezeigte Intensitätsverlauf, welcher durch die Pausen 22 getrennte Impulse 20 aufweist, wird somit durch eine Amplitudenmodulation einer zugrundeliegenden Folge von einzelnen Stoßwellen 24 mit konstanter Frequenz gebildet.

Eine solche Stoßwellenfolge, wie sie in Fig. 3a gezeigt ist, kann in dem Programmmodul 18 voreingestellt werden, so dass dann bei Betätigung des Tasters 8 eine solche Stoßwellenfolge von der Stoßwellenquelle 4 abgegeben wird. Dabei kann die Steuereinrichtung 2 die Stoßwellenfolge so steuern, dass sie diese für eine vorbestimmte Zeitdauer aussendet, welche gegebenenfalls ebenfalls im Programmmodul 18 voreingestellt sein kann. Alternativ kann die Stoßwellenfolge auch manuell, beispielsweise durch nochmalige Betätigung des Tasters 8 beendet werden.

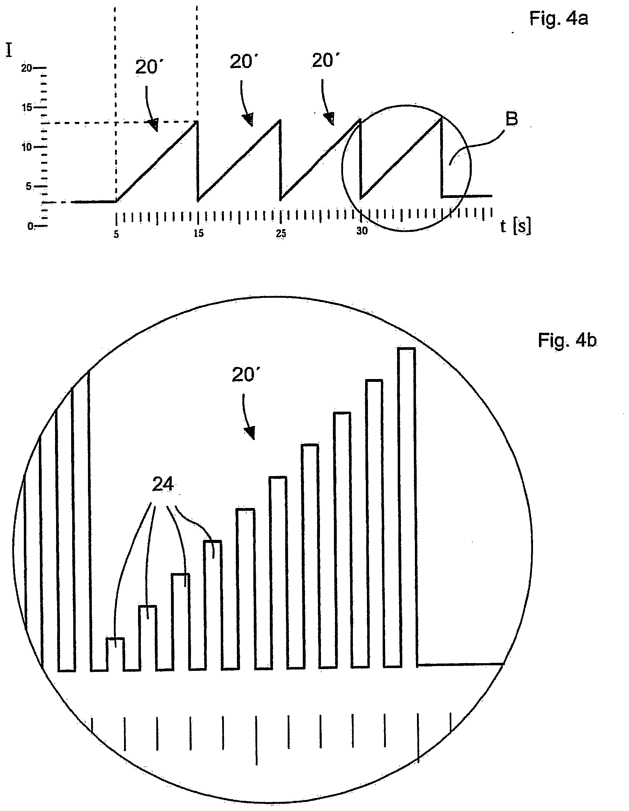

Die Fig. 4a und 4b zeigen ähnlich den Fig. 3a und 3b ein weiteres Beispiel für eine Stoßwellenfolge. Diese Stoßwellenfolge weist einen sägezahnförmigen Kurvenverlauf auf, entsprechend dem Kurvenverlauf in Fig. 2a. Hier schließen sich eine Vielzahl von sägezahnförmigen Impulsen 20' direkt ohne Pausen aneinander an. Auch die einzelnen Impulse 20' werden durch eine Mehrzahl von Einzelstoßwellen 24 erzeugt, welche mit einer festen Frequenz, hier 1 Hertz abgegeben werden. Bei dem Beispiel gemäß Fig. 4a und 4b ändert sich die Intensität der Einzelstoßwellen 24 jedoch nicht lediglich zwischen zwei Intensitätswerten sondern erhöht sich von Einzelstoßwelle 24 zu Einzelstoßwelle 24 in 10 Stufen, wie in Fig. 4b zu sehen ist. Hierdurch ergibt sich der langsame Anstieg der Intensität in dem Impuls 20'. Am Ende des Impulses wird die Intensität dann sprunghaft auf die geringste Intensität, im gezeigten Beispiel die Stufe 3 verringert. Anschließend erhöht sich die Intensität wieder in 10 Stufen über einen Zeitraum von 10 Sekunden auf die Maximalintensität von Stufe 13.

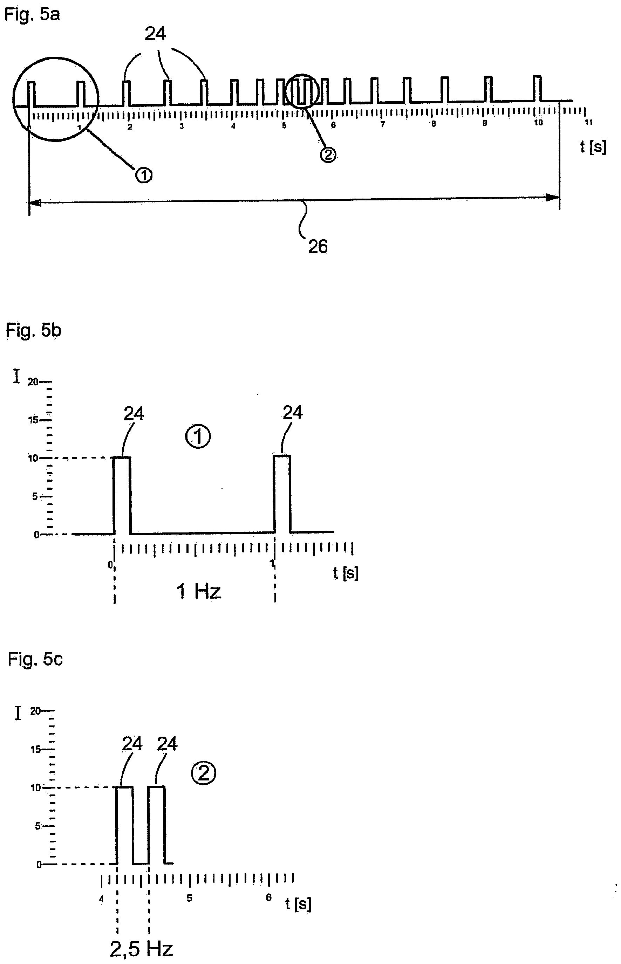

Den Stoßwellenfolgen in den Beispielen von Fig. 3 und Fig. 4 lag eine feste Grundfrequenz für die Stoßwellen 24 zugrunde. Bei der Behandlung kann das Gewebe des Organismus auf die gleichmäßig applizierten Stoßwellen in der Weise reagieren, dass sich ein Gewöhnungseffekt einstellt. Um diesem entgegenzuwirken, kann eine Wobbel-Funktion vorgesehen sein, in der Weise, dass die Frequenz zyklisch variiert wird, was anhand der Figuren 5a bis 5c erläutert wird. Auch in diesen Figuren ist die Intensität I über der Zeit t in Sekunden aufgetragen. Über ein Zeitintervall 26, in diesem Beispiel von 9 Sekunden, wird die Frequenz der Einzelstoßwellen 24 variiert. Zunächst wird mit einer niedrigen Frequenz von 1 Hertz begonnen (siehe Ausschnitt 1 in Fig. 5b). Diese Frequenz wird anschließend auf ein Maximum von 2,5 Hertz erhöht, welches in dem Ausschnitt 2 in Fig. 5c gezeigt ist und anschließend fällt die Frequenz wieder auf die Frequenz von 1 Hertz ab. Es gibt somit zunächst einen Anstieg der Frequenz und dann wieder einen Abfall. Dies wiederholt sich zyklisch mit einer Intervalldauer 26 von in diesem Beispiel 9 Sekunden. Auch eine solche Frequenzänderung kann an dem Programmmodul 18 voreingestellt bzw. in dem Programmmodul 18 gespeichert werden. Nach Auswahl einer solchen Stoßwellenfolge erfolgt dann lediglich eine Aktivierung über den Taster 8, so dass diese Stoßwellenfolge dann für eine vorbestimmte Zeit oder bis zur erneuten Aktivierung des Tasters 8 von der Stoßwellenquelle 4 abgegeben wird. In dem Beispiel gemäß Fig. 5a bis Fig. 5c ist die Intensität der Stoßwellen konstant. Es ist jedoch zu verstehen, dass auch hier eine Variation der Intensität erfolgen könnte, wie es beispielsweise anhand der Fig. 3 und 4 erläutert wurde. Es ist somit auch möglich, Frequenzänderung und Änderung der Pausendauern mit einer Intensitätsänderung zu kombinieren.

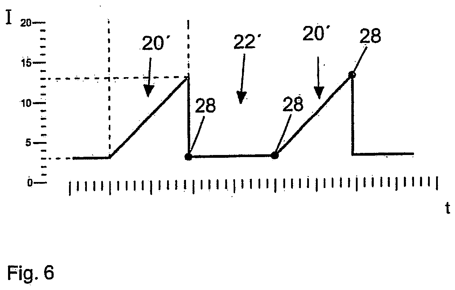

Um den Kurvenverlauf einer Stoßwellenfolge einfach auswählen bzw. voreinstellen zu können, ist bei dieser Ausführungsform vorgesehen, dass die Kurve auf dem Bildschirm 12 dargestellt wird und die Kurve direkt am Bildschirm 12 verändert werden kann. Dies kann durch berührungsempfindliche Ausgestaltung des Bildschirms erfolgen, so dass einzelne Markierungspunkte 28 (Fig. 6) an der Kurve direkt auf dem Bildschirm beispielsweise mit einem Stift oder dem Finger in eine gewünschte Form verschoben werden können. Alternativ kann ein Verschieben beispielsweise auch mittels eines Mauszeigers und mittels einer separaten Eingabeeinrichtung 10 erfolgen. In Fig. 6 ist beispielhaft ein Kurvenverlauf ähnlich dem in Fig. 4a dargestellt, mit dem Unterschied, dass dort zwischen zwei Impulsen 20' jeweils eine Pause 22' vorgesehen ist. Durch Verschieben der Markierungspunkte 28 kann die Kurve zum einen in eine andere Form gebracht werden, zum anderen können die Dauer der Impulse 20' und die Dauer der Pausen 22' auf einfache Weise geändert werden. Auch die Intensität kann durch Verschieben der Punkte 28 leicht verändert werden. So wird eine sehr einfache Bedienung des erfindungsgemäßen Stoßwellengerätes und insbesondere eine sehr einfach Voreinstellung einer definierten Stoßwellenfolge möglich.

### Bezugszeichenliste

- 2: - Steuereinrichtung
- 4: - Stoßwellenquelle
- 6: - Kabel
- 8: - Taster
- 10: - Eingabeeinrichtung
- 12: - Bildschirm
- 14: - HV-Einheit
- 16: - zentrale Steuereinheit (CPU)
- 18: - Programmmodul
- 20, 20': - Impulse
- 22, 22': - Pausen
- 24: - Stoßwellen
- 26: - Zeitintervall
- 28: - Markierungspunkte

- I: - Intensität
- t: - Zeit

## Patentansprüche

1. Stoßwellentherapiegerät für die extrakorporale Stoßwellentherapie mit einer Stoßwellenquelle (4) und einer Steuereinrichtung (2) zur Ansteuerung der Stoßwellenquelle (4),
wobei
die Steuereinrichtung (2) ein Programmmodul (18) aufweist, in welchem zumindest eine vorbestimmte Abfolge einzelner Stoßwellen (24) unterschiedlicher Intensität, Frequenz, Impulsdauer und/oder mit unterschiedlich langen Pausen derart einstellbar ist, dass in dem Programmmodul (18) zumindest eine Abfolge einzelner Stoßwellen (24) gespeichert oder einstellbar ist, deren Frequenz, Intensität (1), Pausen (22) und Impulsdauer (20) zyklisch variieren, und die Stoßwellenquelle (4) von der Steuereinrichtung (2) derart ansteuerbar ist, dass die Steuereinrichtung über das Programmmodul automatisiert die vorgegebene Stoßwellenfolge erzeugt und die Stoßwellenquelle (4) die vorbestimmte Abfolge von Stoßwellen (24) abgibt.

2. Stoßwellentherapiegerät nach Anspruch 1, **dadurch gekennzeichnet, dass** die Steuereinrichtung (2) zur Erzeugung unterschiedlicher Intensitätsverläufe in der Abfolge einzelner Stoßwellen (24) ausgebildet ist.

3. Stoßwellentherapiegerät nach einem der vorangehenden Ansprüche, **dadurch gekennzeichnet, dass** das Programmmodul (18) mit einem Speicher ausgestattet ist, in welchem mehrere vorbestimmte Abfolgen von Stoßwellen (24) gespeichert und/oder speicherbar sind.

4. Stoßwellentherapiegerät nach einem der vorangehenden Ansprüche, **dadurch gekennzeichnet, dass** zumindest eine Eingabeeinrichtung (10) vorgesehen ist, mittels welcher eine vorbestimmte Abfolge von Stoßwellen (24) an dem Programmmodul (18) auswählbar oder einstellbar ist.

5. Stoßwellentherapiegerät nach Anspruch 4, **dadurch gekennzeichnet, dass** die Eingabeeinrichtung (10) einen berührungsempfindlichen Bildschirm (12) aufweist.

6. Stoßwellentherapiegerät nach Anspruch 4 oder 5, **dadurch gekennzeichnet, dass** die Steuereinrichtung (2) derart ausgebildet ist, dass der Kurvenverlauf einer Abfolge von Stoßwellen durch Bewegen von Markierungspunkten (28) einer Kurve auf dem Bildschirm (12) voreinstellbar ist.

7. Stoßwellentherapiegerät nach einem der vorangehenden Ansprüche, **dadurch gekennzeichnet, dass** die Steuereinrichtung (2) einen Lernmodus aufweist, in welchem eine Behandlungssequenz mit einer speziellen Abfolge einzelner Stoßwellen (24) erlernt und in dem Programmmodul (18) gespeichert werden kann.

## Claims

1. A shock wave therapy apparatus for extracorporeal shock wave therapy with a shock wave source (4) and with a control device (2) for activating the shock wave source (4),
wherein
the control device (2) comprises a program module (18), in which at least one predefined sequence of individual shock waves (24) of a different intensity, frequency, impulse duration and/or with differently long pauses can be adjusted in a manner such that at least one sequence of individual shock waves (24) whose frequency, intensity (1), pauses (22) and impulse duration (20) vary in a cyclical manner is stored or adjustable in the program module (18), and the shock wave source (4) can be activated by the control device (2) in a manner such that the control device produces the predefined shockwave sequence in an automated manner via the program module, and the shock wave source (4) emits the predefined sequence of shock waves (24).

2. A shock wave therapy apparatus according to claim 1, **characterised in that** the control device (2) is designed for producing different intensity courses in the sequence of individual shock waves (24).

3. A shock wave therapy apparatus according to one of the preceding claims, **characterised in that** the program module (18) is equipped with a memory, in which several predefined sequences of shock waves (24) are stored and/or storable.

4. A shock wave therapy apparatus according to one of the preceding claims, **characterised in that** at least one input device (10) is provided, by way of which a predefined sequence of shock waves (24) can be selected or adjusted at the program module (18).

5. A shock wave therapy apparatus according to claim 4, **characterised in that** the input device (10) comprises a touch-sensitive screen (12).

6. A shock wave therapy apparatus according to claim 4 or 5, **characterised in that** the control device (2) is designed in a manner such that the curve course of a sequence of shock waves can be preset by way of moving marking points (28) of a curve on the screen (12).

7. A shock wave therapy apparatus according to one of the preceding claims, **characterised in that** the control device (2) comprises a learning mode, in which a treatment sequence with a special sequence of individual shock waves (24) can be learnt and can be stored in the program module (18).

## Revendications

1. Appareil de thérapie à ondes de choc pour la thérapie à ondes de choc extracorporelle, comprenant une source d'ondes de choc (4) et un dispositif de commande (2) pour la commande de la source d'ondes de choc (4),
dans lequel le dispositif de commande (2) comporte un module de programme (18) dans lequel au moins une succession prédéterminée d'ondes de choc (24) individuelles d'intensité, fréquence, durée d'impulsion différentes et/ou à pauses de longueurs différentes est réglable de façon telle qu'au moins une succession d'ondes de choc (24) individuelles est stockée ou réglable dans le module de programme (18), dont la fréquence, l'intensité (1), les pauses (22) et la durée d'impulsion (20) varient de manière cyclique, et dans lequel la source d'ondes de choc (4) peut être commandée par le dispositif de commande (2) de telle sorte que le dispositif de commande, automatisé via le module de programme, génère la succession d'ondes de choc prédéterminée et la source d'ondes de choc (4) émet la succession prédéterminée d'ondes de choc (24).

2. Appareil de thérapie à ondes de choc selon la revendication 1, **caractérisé en ce que** le dispositif de commande (2) est conçu pour produire des courbes d'intensité différentes dans la succession d'ondes de choc individuelles (24).

3. Appareil de thérapie à ondes de choc selon l'une des revendications précédentes, **caractérisé en ce que** le module de programme (18) est pourvu d'une mémoire dans laquelle sont stockées et/ou peuvent être stockées plusieurs successions prédéterminées d'ondes de choc (24).

4. Appareil de thérapie à ondes de choc selon l'une des revendications précédentes, **caractérisé en ce qu'**est prévu au moins un dispositif d'entrée (10) au moyen duquel une succession prédéterminée d'ondes de choc (24) peut être sélectionnée ou réglée sur le module de programme (18).

5. Appareil de thérapie à ondes de choc selon la revendication 4, **caractérisé en ce que** le dispositif d'entrée (10) comporte un écran (12) sensible au toucher.

6. Appareil de thérapie à ondes de choc selon la revendication 4 ou 5, **caractérisé en ce que** le dispositif de commande (2) est conçu de façon telle que l'allure de la courbe d'une succession d'ondes de choc est pré-réglable par le déplacement de points de marquage (28) d'une courbe sur l'écran (12).

7. Appareil de thérapie à ondes de choc selon l'une des revendications précédentes, **caractérisé en ce que** le dispositif de commande (2) comporte un mode d'apprentissage dans lequel une séquence de traitement comprenant une succession spéciale d'ondes de choc (24) individuelles peut être apprise et stockée dans le module de programme (18).
